# EUROPÄISCHE PATENTSCHRIFT

(11) **EP 0 696 312 B1**
(45) Veröffentlichungstag und Bekanntmachung des Hinweises auf die Patenterteilung: **18.11.1998**
(21) Anmeldenummer: 95909748.6
(22) Anmeldetag: 20.02.1995
(51) Int. Cl.: C11B 1/10, C11B 3/16, C11B 1/02, A61K 7/48

(54) **VERFAHREN ZUR GEWINNUNG VON HOCHREINEM EIÖL UND SEINE VERWENDUNG**
PROCESS FOR OBTAINING HIGH-PURITY EGG OIL AND USE THEREOF
PROCEDE DE PRODUCTION D'HUILE A BASE D'OEUF ET SON UTILISATION

(30) Priorität: 21.02.1994 DE 4405486
(43) Veröffentlichungstag der Anmeldung: 14.02.1996
(73) Patentinhaber: NAWROCKI, Werner C., 60320 Frankfurt am Main (DE)
(72) Erfinder: NAWROCKI, Werner C., 60320 Frankfurt am Main (DE)
(74) Vertreter: Jung, Elisabeth, Dr.
(86) Internationale Anmeldenummer: EP9500609
(87) Internationale Veröffentlichungsnummer: WO9522590

(56) Entgegenhaltungen:
- DE-A- 4 200 678
- GB-A- 2 072 016
- US-A- 4 219 544
- US-A- 4 219 585
- US-A- 5 028 449
- DATABASE WPI Week 9244 Derwent Publications Ltd., London, GB; AN 92-364471 & SU,A,1 701 737 (KOMPLEKS POULTRY IND RES PRODN ASSOC) , 30.Dezember 1991
- DATABASE WPI Week 91 Derwent Publications Ltd., London, GB; Class 27, AN 91-199441 & SU,A,1 604 375 (DNEPR CHEM TECHN INST) , 7.November 1990
- DATABASE WPI Week 9101 Derwent Publications Ltd., London, GB; AN 91-004849 & JP,A,02 283 263 (KOBAYASHI H) , 20.November 1990
- DATABASE WPI Week 8519 Derwent Publications Ltd., London, GB; AN 85-113564 & JP,A,60 055 095 (NAKAZONO S) , 29.März 1985
- PATENT ABSTRACTS OF JAPAN vol. 14 no. 456 (C-0765) ,2.Oktober 1990 & JP,A,02 180994 (RANSUTAA KK) 13.Juli 1990,
- COSMETICS AND PERFUMERY, Bd. 88,Nr. 1, 1973 US, Seiten 31-37, DANIEL MELNICK 'Potential value of egg oil in cosmetic products'

## Beschreibung

Einen erheblicher Nachteil, der vielen gebräuchlichen, nach dem gegenwärtigen Stand der Technik industriell hergestellten Medikamenten zur Behandlung von Hautverbrennungen, insbesondere Sonnenbrand, gemeinsam ist, stellt die mangelnde Zersetzungsstabilität der verwendeten Wirkstoffe dar. Gleiches gilt für zahlreiche im Handel erhältliche kosmetische Präparate zur Regeneration der Haut.
Daraus resultiert in der Regel die Notwendigkeit, diesen Erzeugnissen im Zuge der Produktion Konservierungsstoffe zuzusetzen, welche eine langfristige Lagerung des Produkts erleichtern oder überhaupt erst ermöglichen.
Das Potential an schwer kalkulierbaren, häufig kaum einzugrenzenden oder gar vorhersehbaren Nebenwirkungen, das von derartigen, konservierenden Additiven ausgeht, stellt allerdings oft für den Anwender der Medikamente einen ausgesprochen unerwünschten Risikofaktor dar. Vor dem Hintergrund des zunehmenden Auftretens zivilisationsbedingter Hautschäden, und insbesondere in Anbetracht einer eklatant gesteigerten Häufigkeit des Auftretens von Hautallergien aller Art, erscheint es wünschenswert, das Spektrum möglicher Gesundheitsgefährdungen, das von konservierenden Zusätzen in Medikamenten der eingangs erwähnten Art ausgeht, wirksam einzugrenzen, bzw., soweit möglich, gänzlich zu eliminieren.

Die vorliegende Erfindung beschreibt ein Verfahren zur Herstellung eines hochreinen Eiöls, welches aus dem Eigelb von Vögeln oder Reptilien gewonnen werden kann. Das Produkt kann als naturreines Mittel zur Therapie von Hautverbrennungen aller Art, einschliesslich Sonnenbrand, sowie auch als Wirkstoff zum Regenerieren erkrankter oder infolge besonderer Beanspruchung angegriffener Hautflächen eingesetzt werden. Dabei gilt es zu betonen, daß die Erfindung, welche einen mehrstufigen Reinigungsprozess einschliesst, am Ende zu einem hochreinen Produkt führt, welches auf jegliche konservierende Additive zur Erhöhung der Lagerungsstabilität vollständig verzichten kann. Da das Produkt nicht auf stabilisierende Konservierungsstoffe angewiesen ist, können auch alle oben beschriebenen Nebenwirkungen und Gesundheitsrisiken vollständig ausgeschlossen werden.
Das erfindungsgemäße Verfahren umfasst eine spezielle Kombination von Verfahrensschritten, welche erst in ihrer Gesamtheit und in der angegebenen Abfolge zu dem angestrebten Ergebnis eines hochreinen Eiöls führen.
Das Verfahren ist demgemäß gekennzeichnet durch die folgenden Verfahrensschritte:
a) Trocknen des Eigelbs bei Temperaturen bis 90 °C und Zerkleinern zu einem pulverförmigen Produkt (A),
b) Extrahieren des Produkts (A) aus Stufe (a) während eines Zeitraums von drei bis sieben Tagen mit einem fettlösenden Extraktionsmittel,
c) langsames Abdestillieren des Extraktionsmittels unter Bildung eines dickflüssigen Rückstandes (B),
d) Altern des Rückstandes (B) aus Stufe (c) bei Umgebungstemperatur während eines Zeitraumes bis zu 10 Stunden, vorzugsweise während fünf bis sieben Stunden,
e) weiteres Altern des Rückstandes (B) bei einer Temperatur zwischen 7 und 12 °C während eines Zeitraumes bis zu 24 Stunden, bis eine deutliche Phasentrennung auftritt,
f) Abtrennen der niedrigerviskosen Phase (C), die sich bei der Phasentrennung in Stufe (e) gebildet hat,
g) Zentrifugieren des niedrigerviskosen Produkts (C) aus Stufe (f) während 20 bis 40 Minuten bei einer Geschwindigkeit von bis zu 7000 Umdrehungen pro Minute, und Abtrennen der niedrigerviskosen Phase (D),
h) Mischen des Produkts (D) aus Stufe (g) mit entmineralisiertem Wasser im Volumenverhältnis von 1:2 bis 1:5 und Erhitzen der Mischung während eines Zeitraumes von 30 bis 90 Minuten auf eine Temperatur von 90 bis 120 °C, vorzugsweise auf 95 bis 100 °C,
i) Abkühlung der wäßrigen Mischung von Stufe (h) auf Umgebungstemperatur und Abtrennung des niedrigerviskosen Anteils (E),
j) Zentrifugieren des Produkts (E) aus Stufe (i) während 20 bis 40 Minuten, bei einer Geschwindigkeit von bis zu 7000 Umdrehungen pro Minute und Abtrennung der niedrigerviskosen Phase (F),
k) Halten des Produkts (F) aus Stufe (j) bei Temperaturen von 7 bis 12 °C während eines Zeitraums bis zu 24 Stunden, bis eine deutliche Phasentrennung auftritt, und Abtrennen der niedrigerviskosen Phase (G),
l) Zentrifugieren des Produkts (G) aus Stufe (k) während 20 bis 40 Minuten bei einer Geschwindigkeit von bis zu 7000 Umdrehungen pro Minute und Abtrennen der niedrigerviskosen Phase (H),
m) gegebenenfalls Wiederholung der Maßnahmen der Stufen (h) bis (1) oder (j) bis (1) mit dem Produkt (H) der Stufe (1).

Für das Verfahren gemäß der vorliegenden Erfindung wird das Eigelb von Vögeln wie Hühner, Enten, Strausse, etc., oder auch alternativ von Reptilien, wie beispielsweise Schildkröten als Ausgangsmaterial eingesetzt.
Dieses Ausgangsmaterial wird in einem ersten Verfahrensschritt (a) bei Temperaturen bis maximal 90 °C, vorzugsweise zwischen 50 °C und 70 °C, getrocknet. In industriellem Maßstab wird die Herstellung von getrocknetem Eigelb in der Regel in sogenannten Falltürmen durchgeführt; es eignen sich hierfür aber auch Trockenöfen mit z. B. zirkulierender Luft. Das getrocknete Eigelb wird auf eine mittlere Korngrösse zwischen z.B. 0,3 und 1,0 mm zerkleinert; beispielsweise wird es durch ein Sieb in Form eines feinmaschigen Metalldrahtnetzes (10 x 10 bis 30 x 30 Maschen pro cm²) gepresst. Dieses getrocknete Eigelbpulver (A) wird anschließend einem Extraktionsverfahren (b) unter Verwendung eines fettlösenden Extraktionsmittels unterzogen, welches drei bis sieben Tage dauern soll, vorzugsweise aber vier bis fünf Tage dauert. Als Extraktionsmittel kommen vorzugsweise
- aliphatische Alkohole (mit einem bis fünf, vorzugsweise mit einem bis vier Kohlenstoffatomen pro Molekül, günstigerweise Methanol oder Ethanol) und/oder
- aliphatische Ketone (mit bis zu fünf Kohlenstoffatomen im Molekül, günstigerweise Aceton), und/oder
- Freone, und/oder
- aliphatische Ether (mit bis zu sieben Kohlenstoffatomen im Molekül, insbesondere Dialkylether, wie Diethylether, sowie zyklische Ether, insbesondere Tetrahydrofuran), und/oder
- aliphatische Ester, vorzugsweise Essigsäureethylester, und/oder alternativ
- Kohlenstoffdioxid
in Betracht.
Die verwendete Extraktionstechnik kann aus verschiedenen, konventionellen Labortechniken ausgewählt werden, wobei alternativ auch eine Kombination mehrerer der hier nachfolgend aufgeführten Methoden verwendet werden kann:
- Maceration bei Drücken, die entweder niedriger oder mäßig höher als der Atmosphärendruck sein können, und / oder
- Extraktion nach dem Soxleth-Verfahren, und / oder
- Druckextraktion mit Kohlenstoffdioxid, und / oder
- Mildes Kochen unter Rückfluß.

Nach der Extraktionsbehandlung wird das verwendete Extraktionsmittel in der Stufe (c) langsam abdestilliert, wobei ein dickflüssiger Rückstand (B) verbleibt, welcher als Ausgangsmaterial für die weiteren Verfahrensschritte herangezogen wird.
Danach erfolgt ein in zwei Stufen (d und e) vorgenommenes Altern dieses dickflüssigen Rückstandes (B), nämlich zunächst bei Umgebungstemperatur während eines Zeitraumes von bis zu 10 Stunden, vorzugsweise während fünf bis sieben Stunden, sowie anschließend bei einer Temperatur von 7 bis 12 °C während eines Zeitraums bis zu 24 Stunden, und zwar, bis eine deutlich erkennbare Phasentrennung auftritt.
Die während des Alterns gebildete, niedrigerviskose Phase (C) wird in Stufe (f) abgetrennt, z. B. entweder durch Abdekantation oder mit Hilfe eines Separators, und während eines Zeitraumes von 20 bis 40 Minuten, vorzugsweise für 30 Minuten, mit einer Geschwindigkeit von bis zu 7000 Umdrehungen pro Minute, z.B. mit einer Geschwindigkeit von 900 bis 1200 Umdrehungen pro Minute und vorzugsweise von 4000 bis 7000 Umdrehungen pro Minute, zentrifugiert.

Daraus resultiert eine Phasentrennung, woraufhin die gebildete, niedrigerviskose Phase (D), z. B. durch Abdekantation oder mit Hilfe eines Separators, abgetrennt und weiterverarbeitet wird. Die höherviskose Phase wird verworfen.

Die erhaltene, niedrigerviskose Phase (D) wird nun in Stufe (h) mit entmineralisiertem Wasser vermischt, wobei ein Volumenverhältnis zwischen 1:2 und 1:5 gewählt wird. Die erhaltene Mischung wird danach während eines Zeitraumes von 30 bis 90 Minuten, vorzugsweise während 60 Minuten, auf eine Temperatur von 90 bis 120 °C, vorzugsweise auf 95 bis 100 °C erhitzt. Nach Abkühlung der Mischung auf Umgebungstemperatur tritt eine Phasentrennung auf, woraufhin der niedrigerviskose Anteil (E), z. B. durch Dekantation und/oder mit Hilfe eines Scheidetrichters, abgetrennt wird (Stufe (i)). Der höherviskose Anteil wird verworfen.

Der erhaltene, niedrigerviskose Anteil (E) wird in Stufe (j) mit einer Geschwindigkeit von bis zu 7000 Umdrehungen pro Minute, z.B. mit einer Geschwindigkeit von 900 bis 1200 Umdrehungen pro Minute und vorzugsweise von 4000 bis 7000 Umdrehungen pro Minute, während eines Zeitraumes zwischen 20 und 40 Minuten, vorzugsweise von 30 Minuten, zentrifugiert, was wiederum zu einer Phasentrennung führt. Der niedrigerviskose Anteil (F) wird z. B. abdekantiert oder mittels eines Separators abgetrennt. Die höherviskose Phase wird verworfen.

Der niedrigerviskose Anteil (F) wird in Stufe (k) für einen Zeitraum von maximal 24 Stunden bei einer Temperatur von 7 bis 12 °C gehalten, bis eine erneute, deutliche Phasentrennung auftritt. Die niedrigerviskose Phase (G) wird abgetrennt, z.B. durch Dekantieren, die gebildete, höherviskose Phase verworfen. Die niedrigerviskose Phase (G) wird während eines Zeitraumes zwischen 20 und 40 Minuten, vorzugsweise während 30 Minuten, mit einer Geschwindigkeit von bis zu 7000 Umdrehungen pro Minute, z.B. mit einer Geschwindigkeit von 900 bis 1200 Umdrehungen pro Minute und vorzugsweise von 4000 bis 7000 Umdrehungen pro Minute, zentrifugiert (Stufe (1)). Die dabei gebildete, niedrigerviskose Phase (H) stellt das Endprodukt dar.

Gegebenenfalls können auch die Verfahrensschritte entweder ab dem Vermischen von (D) mit entmineralisiertem Wasser bis zur Zentrifugation von (G) (Stufen (h) bis (1)), oder ab der Zentrifugation von (E) bis zur Zentrifugation von (G) (Stufen (j) bis (1)), wiederholt werden, um ein besonders reines Endprodukt (H) der vorliegenden Erfindung zu erhalten.

Das Endprodukt (H) ist ein bewegliches Öl von, je nach verwendetem Ausgangsmaterial, gelber bis rötlicher Farbe, welches ohne Zusatz von Konservierungsstoffen gelagert werden kann.

Die vorliegende Erfindung wird jetzt mit Hilfe eines repräsentativen Beispiels näher erläutert.

### BEISPIEL 1:

(a) Das Eigelb von 10 frischen Hühnereiern wurde bei einer Temperatur von 60 °C in einem Labortrockenofen getrocknet. Das getrocknete Eigelb wurde danach durch ein handelsübliches, halbkugelförmiges Haushaltssieb aus rostfreiem Stahldraht (20 x 20 Maschen pro cm²) gepresst und dadurch auf eine mittlere Korngrösse von ca. 0,5 mm zerkleinert.
(b) Dieses getrocknete Eigelbpulver wurde anschließend in einer laborüblichen Soxleth-Apparatur unter Verwendung von 250 ml Aceton p.a. einer fünftägigen Extraktionsbehandlung unterzogen.
(c) Nach der Extraktionsbehandlung wurde das verwendete Aceton unter Verwendung eines Rotationsverdampfers im leichten Vakuum abdestilliert.
(d) + (e) Dabei verblieb ein dickflüssiger Rückstand, welcher danach für sechs Stunden bei Umgebungstemperatur und anschließend für 24 Stunden in einem temperierten Kühlraum bei 10 °C gealtert wurde. Gegen Ende des Alterns wurde eine deutliche Phasentrennung beobachtet.
(f) Daraufhin wurde die niedrigerviskose Phase abdekantiert und die höherviskose verworfen.
(g) Der niedrigerviskose Anteil wurde jetzt während eines Zeitraumes von 30 Minuten mit einer Geschwindigkeit von 1100 Umdrehungen pro Minute zentrifugiert.
   Es fand eine Phasentrennung statt, woraufhin die entstandene, niedrigerviskose Phase abdekantiert und die höherviskose Phase verworfen wurde.
(h) + (i) Die erhaltene, niedrigerviskose Phase wurde nun in einem Volumenverhältnis von 1:4 mit entmineralisiertem Wasser versetzt.
   Diese Mischung wurde während eines Zeitraumes von 60 Minuten auf eine Temperatur von 96 °C erhitzt. Nach Abkühlung der Mischung auf Umgebungstemperatur trat eine Phasentrennung auf, woraufhin der niedrigerviskose Anteil durch Dekantation abgetrennt wurde. Der höherviskose Anteil wurde verworfen.
(j) Der erhaltene, niedrigerviskose Anteil wurde mit einer Geschwindigkeit von 1100 Umdrehungen pro Minute während eines Zeitraumes von 30 Minuten zentrifugiert, was zu einer Phasentrennung führte. Die höherviskose Phase wurde verworfen.
(k) Der abdekantierte, niedrigerviskose Anteil wurde für einen Zeitraum von 24 Stunden in einem temperierten Kühlraum bei einer Temperatur von 10 °C gehalten, wobei eine deutliche Phasentrennung auftrat. Die entstandene, höherviskose Phase wurde verworfen und die niedrigerviskose Phase abdekantiert.
(l) Letztere wurde anschließend während eines Zeitraumes von 30 Minuten mit einer Geschwindigkeit von 1100 Umdrehungen pro Minute zentrifugiert.
   Die dabei gebildete, niedrigerviskose Phase war das Endprodukt der vorliegenden Erfindung, ein leicht bewegliches Öl von gelblicher Farbe, welches ohne Zusatz von Konservierungsstoffen stabil gelagert werden konnte.

### BEISPIEL 2:

Die Verfahrensfolge von Beispiel 1 wurde mit dem Eigelb von 10 frischen Hühnereiern wiederholt, wobei das Zentrifugieren im Rahmen der Verfahrensschritte (g), (j) und (1) jedoch mit einer Geschwindigkeit von 6000 Umdrehungen pro Minute durchgeführt wurde.

## Patentansprüche

1. Ein Verfahren zur Gewinnung von hochreinem Eiöl aus dem Eigelb von Vögeln oder Reptilien, gekennzeichnet durch die folgenden Verfahrensschritte:
a) Trocknen des Eigelbs bei Temperaturen bis 90 °C und Zerkleinern zu einem pulverförmigen Produkt (A),
b) Extrahieren des Produkts (A) aus Stufe (a) während eines Zeitraums von drei bis sieben Tagen mit einem fettlösenden Extraktionsmittel,
c) langsames Abdestillieren des Extraktionsmittels unter Bildung eines dickflüssigen Rückstandes (B),
d) Altern des Rückstandes (B) aus Stufe (c) bei Umgebungstemperatur während eines Zeitraumes bis zu 10 Stunden, vorzugsweise während fünf bis sieben Stunden,
e) weiteres Altern des Rückstandes (B) bei einer Temperatur zwischen 7 und 12 °C während eines Zeitraumes bis zu 24 Stunden, bis eine deutliche Phasentrennung auftritt,
f) Abtrennen der niedrigerviskosen Phase (C), die sich bei der Phasentrennung in Stufe (e) gebildet hat,
g) Zentrifugieren des niedrigerviskosen Produkts (C) aus Stufe (f) während 20 bis 40 Minuten bei einer Geschwindigkeit von bis zu 7000 Umdrehungen pro Minute, und Abtrennen der niedrigerviskosen Phase (D),
h) Mischen des Produkts (D) aus Stufe (g) mit entmineralisiertem Wasser im Volumenverhältnis von 1:2 bis 1:5 und Erhitzen der Mischung während eines Zeitraumes von 30 bis 90 Minuten auf eine Temperatur von 90 bis 120 °C, vorzugsweise auf 95 bis 100 °C,
i) Abkühlung der wäßrigen Mischung von Stufe (h) auf Umgebungstemperatur und Abtrennung des niedrigerviskosen Anteils (E),
j) Zentrifugieren des Produkts (E) aus Stufe (i) während 20 bis 40 Minuten, bei einer Geschwindigkeit von bis zu 7000 Umdrehungen pro Minute und Abtrennung der niedrigerviskosen Phase (F),
k) Halten des Produkts (F) aus Stufe (j) bei Temperaturen von 7 bis 12 °C während eines Zeitraums bis zu 24 Stunden, bis eine deutliche Phasentrennung auftritt, und Abtrennen der niedrigerviskosen Phase (G),
l) Zentrifugieren des Produkts (G) aus Stufe (k) während 20 bis 40 Minuten bei einer Geschwindigkeit von bis zu 7000 Umdrehungen pro Minute und Abtrennen der niedrigerviskosen Phase (H),
m) gegebenenfalls Wiederholung der Maßnahmen der Stufen (h) bis (1) oder (j) bis (1) mit dem Produkt (H) der Stufe (1).

2. Ein Verfahren wie in Anspruch 1 beansprucht, dadurch gekennzeichnet, daß das Eigelb bei einer Temperatur zwischen 50 °C und 70 °C getrocknet wird.

3. Ein Verfahren nach Anspruch 1 und 2, dadurch gekennzeichnet, daß das zur Extraktion von (A) in Stufe (b) verwendete Extraktionsmittel aus den nachfolgenden Substanzen, bzw. Substanzgruppen aa) bis ff) ausgewählt wird:
aa) aliphatische Alkohole mit einem bis fünf, vorzugsweise einem bis vier Kohlenstoffatomen im Molekül, vorzugsweise Methanol oder Ethanol,
bb) aliphatische Ketone mit bis zu fünf Kohlenstoffatomen im Molekül, vorzugsweise Aceton,
cc) Freone,
dd) aliphatische Ether mit bis zu sieben Kohlenstoffatomen im Molekül, insbesondere Dialkylether, wie Diethylether, sowie zyklische Ether, insbesondere Tetrahydrofuran,
ee)aliphatische Ester, insbesondere Essigsäureethylester,
ff) CO₂.

4. Ein Verfahren nach Anspruch 1 bis 3, dadurch gekennzeichnet, daß die zur Extraktion von (A) in Stufe (b) verwendete Extraktionstechnik aus den nachfolgend genannten Extraktionstechniken a1) bis d1) ausgewählt wird:
a1) Maceration bei Drücken zwischen Vakuum und mäßigem Überdruck, bei Raumtemperatur oder bei erhöhter Temperatur, und / oder
b1) Extraktion nach dem Soxleth-Verfahren, und / oder
c1) Druckextraktion mit CO₂, und / oder
d1) Mildes Kochen unter Rückfluß.

5. Ein Verfahren nach Anspruch 1 bis 4, dadurch gekennzeichnet, daß die Extraktion von (A) in Stufe (b) vier bis fünf Tage lang durchgeführt wird.

6. Ein Verfahren nach Anspruch 1 bis 5, dadurch gekennzeichnet, daß als Trennverfahren zum Abtrennen der jeweils niedrigerviskosen Phasen (C) bis (H) entweder eine Dekantation vorgenommen oder ein Separator verwendet wird.

7. Ein Verfahren nach Anspruch 1 bis 6, dadurch gekennzeichnet, daß das Zentrifugieren von (C), (E) und (G) in den Stufen (g), (j) und (1) jeweils während eines Zeitraums von 30 Minuten vorgenommen wird.

8. Ein Verfahren nach Anspruch 1 bis 7, dadurch gekennzeichnet, daß das Zentrifugieren von (C) in Stufe (g), von (E) in Stufe (j) und von (G) in Stufe (1) bei einer Geschwindigkeit von 4000 bis 7000 Umdrehungen pro Minute vorgenommen wird.

9. Ein Verfahren nach Anspruch 1 bis 7, dadurch gekennzeichnet, daß das Zentrifugieren von (C) in Stufe (g), von (E) in Stufe (j) und von (G) in Stufe (1) bei einer Geschwindigkeit von 900 bis 1200 Umdrehungen pro Minute vorgenommen wird.

10. Ein Verfahren nach Anspruch 1 bis 9, dadurch gekennzeichnet, daß die in Stufe (h) hergestellte Mischung aus Produkt (D) und entmineralisiertem Wasser vorzugsweise 60 Minuten lang erhitzt wird.

11. Verwendung des hochgereinigten Eiöls, hergestellt gemäß den Ansprüchen 1 bis 10, als Mittel zum Regenerieren der Haut in Kosmetika.

## Claims

1. A process for obtaining ultrapure egg oil from avian or reptilian egg yolk, characterized by the following process steps:
a) drying the egg yolk at temperatures up to 90°C and comminuting the dried egg yolk to give a pulverulent product (A),
b) extracting the product (A) obtained in step (a) over a period of three to seven days using a fat-dissolving extractant,
c) slowly distilling off the extractant to obtain a viscous residue (B),
d) ageing the residue (B) obtained in step (c) at ambient temperature over a period of up to 10 hours, preferably for five to seven hours,
e) further ageing the residue (B) at a temperature of between 7 and 12°C over a period of up to 24 hours until distinct phase separation takes place,
f) separating off the less viscous phase (C) formed upon phase separation in step (e),
g) centrifuging the less viscous product (C) obtained in step (f) for 20 to 40 minutes at a speed of up to 7,000 revolutions per minute, and separating off the less viscous phase (D),
h) mixing the product (D) obtained in step (g) with demineralized water in a ratio of 1:2 to 1:5 by volume, and heating the mixture over a period of 30 to 90 minutes at a temperature of 90 to 120°C, preferably 95 to 100°C,
i) cooling the aqueous mixture obtained in step (h) to ambient temperature, and separating off the less viscous component (E),
j) centrifuging the product (E) obtained in step (i) for 20 to 40 minutes at a speed of up to 7,000 revolutions per minute, and separating off the less viscous phase (F),
k) holding the product (F) obtained in step (j) at temperatures from 7 to 12°C over a period of up to 24 hours until distinct phase separation takes place, and separating off the less viscous phase (G),
l) centrifuging the product (G) obtained in step (k) for 20 to 40 minutes at a speed of up to 7,000 revolutions per minute, and separating off the less viscous phase (H),
m) if appropriate, repeating the measures of steps (h) to (1) or (j) to (1) using the product (H) obtained in step (1).

2. A process as claimed in Claim 1, characterized in that the egg yolk is dried at a temperature of between 50°C and 70°C.

3. A process according to Claims 1 and 2, characterized in that the extractant used for the extraction of (A) in step (b) is selected from the following substances or substance groups aa) to ff):
aa) aliphatic alcohols having one to five, preferably one to four carbon atoms per molecule, preferably methanol or ethanol,
bb) aliphatic ketones having up to five carbon atoms per molecule, preferably acetone,
cc) Freons,
dd) aliphatic ethers having up to seven carbon atoms per molecule, in particular dialkyl ethers, such as diethyl ether, and cyclic ethers, in particular tetrahydrofuran,
ee) aliphatic esters, in particular ethyl acetate, and
ff) CO₂.

4. A process according to Claims 1 to 3, characterized in that the extraction technique used for the extraction of (A) in step (b) is selected from amongst the extraction techniques a1) to d1) mentioned below:
a1) maceration under pressures between a vacuum and a moderate superatmospheric pressure, at room temperature or elevated temperature, and/or
b1) extraction by the Soxleth [sic] process, and/or
c1) extraction under pressure using CO₂, and/or
d1) refluxing under mild conditions.

5. A process according to Claims 1 to 4, characterized in that the extraction of (A) in step (b) is carried out for four to five days.

6. A process according to Claims 1 to 5, characterized in that the separating method for separating off phases (C) to (H), which are in each case the less viscous ones, is either carried out by decanting or by using a separator.

7. A process according to Claims 1 to 6, characterized in that the centrifuging of (C), (E) and (G) in steps (g), (j) and (1) is in each case carried out over a period of 30 minutes.

8. A process according to Claims 1 to 7, characterized in that the centrifuging of (C) in step (g), of (E) in step (j) and of (G) in step (1) is carried out at a speed of 4,000 to 7,000 revolutions per minute.

9. A process according to Claims 1 to 7, characterized in that the centrifuging of (C) in step (g), of (E) in step (j) and of (G) in step (l) is carried out at a speed of 900 to 1,200 revolutions per minute.

10. A process according to Claims 1 to 9, characterized in that the mixture of product (D) and demineralized water, which is prepared in step (h), is heated for preferably 60 minutes.

11. Use of the ultrapure egg oil prepared in accordance with Claims 1 to 10 as a product for regenerating the skin in cosmetic compositions.

## Revendications

1. Procédé d'obtention d'huile d'oeuf de haute pureté à partir du jaune d'oeuf d'oiseaux ou de reptiles, caractérisé par les opérations de base suivantes :
a) séchage du jaune d'oeuf à des températures pouvant atteindre 90°C et fractionnement en un produit pulvérulent (A),
b) extraction du produit (A) de l'étape (a) pendant une durée de 3 à 7 jours avec un agent d'extraction dissolvant les graisses,
c) élimination lente de l'agent d'extraction par distillation avec formation d'un résidu visqueux (B),
d) vieillissement du résidu (B) de l'étape (c) à la température ambiante pendant une durée pouvant atteindre 10 h, de préférence pendant 5 à 7 h,
e) vieillissement supplémentaire du résidu (B) à une température comprise entre 7 et 12°C pendant une durée pouvant atteindre 24 h, jusqu'à ce qu'une séparation de phases nette se produise,
f) séparation de la phase moins visqueuse (C) qui s'est formée lors de la séparation de phases dans l'étape (e),
g) centrifugation du produit moins visqueux (C) de l'étape (f) pendant 20 à 40 min à une vitesse pouvant atteindre 7 000 tr/min, et séparation de la phase moins visqueuse (D),
h) mélange du produit (D) de l'étape (g) avec de l'eau déminéralisée dans le rapport volumique de 1:2 à 1:5 et chauffage du mélange pendant une durée de 30 à 90 min à une température de 90 à 120°C, de préférence de 95 à 100°C,
i) refroidissement du mélange aqueux de l'étape (h) à la température ambiante et séparation de la partie moins visqueuse (E),
j) centrifugation du produit (E) de l'étape (i) pendant 20 à 40 min, à une vitesse pouvant atteindre 7 000 tr/min, et séparation de la phase moins visqueuse (F),
k) maintien du produit (F) de l'étape (j) à des températures de 7 à 12°C pendant une durée pouvant atteindre 24 h, jusqu'à ce qu'une séparation de phases nette se produise, et séparation de la phase moins visqueuse (G),
l) centrifugation du produit (G) de l'étape (k) pendant 20 à 40 min à une vitesse pouvant atteindre 7 000 tr/min et séparation de la phase moins visqueuse (H),
m) le cas échéant, répétition des mesures des étapes (h) à (I) ou (j) à (I) avec le produit (H) de l'étape (l).

2. Procédé selon la revendication 1, caractérisé en ce que le jaune d'oeuf est séché à une température comprise entre 50°C et 70°C.

3. Procédé selon les revendications 1 et 2, caractérisé en ce que l'agent d'extraction employé pour l'extraction de (A) dans l'étape (b) est choisi parmi les substances ou groupes de substances aa) à ff) suivants :
aa) les alcools aliphatiques ayant 1 à 5, de préférence 1 à 4 atomes de carbone dans la molécule, de préférence le méthanol ou l'éthanol,
bb) les cétones aliphatiques ayant jusqu'à 5 atomes de carbone dans la molécule, de préférence l'acétone,
cc) les Fréons,
dd) les éthers aliphatiques ayant jusqu'à 7 atomes de carbone dans la molécule, en particulier les dialkyléthers comme le diéthyléther, ainsi que les éthers cycliques, en particulier le tétrahydrofurane,
ee) les esters aliphatiques, en particulier l'acétate d'éthyle,
ff) CO₂.

4. Procédé selon les revendications 1 à 3, caractérisé en ce que la technique d'extraction employée pour l'extraction de (A) dans l'étape (b) est choisie parmi les techniques d'extraction a1) à d1) citées dans la suite :
a1) macération à des pressions comprises entre le vide et une légère surpression, à la température ambiante ou à température augmentée, et/ou
b1) extraction selon le procédé Soxleth, et/ou
c1) extraction sous pression avec CO₂, et/ou
d1) ébullition douce à reflux.

5. Procédé selon les revendications 1 à 4, caractérisé en ce que l'extraction de (A) dans l'étape (b) est réalisée pendant 4 à 5 jours.

6. Procédé selon les revendications 1 à 5, caractérisé en ce qu'on réalise une décantation ou on utilise un séparateur comme procédé de séparation pour la séparation des phases moins visqueuses (C) à (H).

7. Procédé selon les revendications 1 à 6, caractérisé en ce que la centrifugation de (C), (E) et (G) dans les étapes (g), (j) et (I) est réalisée à chaque fois pendant une durée de 30 min.

8. Procédé selon les revendications 1 à 7, caractérisé en ce que la centrifugation de (C) dans l'étape (g), de (E) dans l'étape (j) et de (G) dans l'étape (I) est réalisée à une vitesse de 4 000 à 7 000 tr/min.

9. Procédé selon les revendications 1 à 7, caractérisé en ce que la centrifugation de (C) dans l'étape (g), de (E) dans l'étape (j) et de (G) dans l'étape (l) est réalisée à une vitcsse de 900 à 1 200 tr/min.

10. Procédé selon les revendications 1 à 9, caractérisé en ce que le mélange de produit (D) et d'eau déminéralisée préparé dans l'étape (h) est chauffé de préférence pendant 60 min.

11. Utilisation de l'huile d'oeuf hautement purifiée préparée selon les revendications 1 à 10 comme agent pour la régénération de la peau dans des cosmétiques.
